# EUROPEAN PATENT SPECIFICATION

(11) **EP 0 986 985 B1**
(45) Date of publication and mention of the grant of the patent: **03.12.2003**
(21) Application number: 99116496.3
(22) Date of filing: 23.08.1999
(51) Int. Cl.: A47K 3/00, A61M 21/00, A61H 33/00

(54) **Method and apparatus for bringing about beneficial effects on the human body**
Verfahren und Vorrichtung zur positiven Beeinflussung des menschlichen Körpers
Procédé et dispositif pour apporter des effets bénéfiques au corps humain

(30) Priority: 15.09.1998 IT PN980066
(43) Date of publication of application: 22.03.2000
(73) Proprietor: DOMINO S.p.A., 33097 Spilimbergo, Pordenone (IT)
(72) Inventor: Colussi, Lucio, 33072 Casarsa, Pordenone (IT)
(74) Representative: Giugni, Valter

(56) References cited:
- FR-A- 2 701 842
- US-A- 3 585 991
- US-A- 4 640 266
- US-A- 5 604 940
- PATENT ABSTRACTS OF JAPAN vol. 15, no. 249 (M-1128), 25 June 1991 (1991-06-25) & JP 03 079953 A (MATSUSHITA ELECTRIC IND CO LTD), 4 April 1991 (1991-04-04)

## Description

The present invention refers to a method and an apparatus for the treatment of the human body by using a combination of external actions so as to bring about definite sensations of well-being that get persons involved both physically and mentally. Such a treatment, however, is not to be regarded as a medical or therapeutical one.

Particularly suitable for implementing the invention is the ambient of the bathroom, since the devices needed for such an implementation can be applied to a bathtub or a shower booth, ie. fittings that are normally to be found in such an ambient.

The beneficial properties of the so-called aromatherapy, ie. the use of essential oils of a number of different plants to therapeutical purposes, has been known and recognized since ancient times. In fact, inhaling and absorbing such substances through the skin bring about significant effects that prove particularly beneficial against traditional psychosomatic pathologies. Nowadays, aromatherapy is known to be quite effective in combating stress, which represents what may be well regarded as one of the major factors endangering the energetic equilibrium of a person. Aromas and fragrances introduced in the bath water, or better in the jets of a whirpool, intensify their effect since essences are able to more easily seep through the pores of the skin and the respiratory tract. To this purpose, the stimulation of olfaction turns out to be very important since it induces deeper breathing.

Regardless of other factors, various types of music are also largely known to have an influence, although in different manners and to differing extents, on the sensitiveness and, therefore, the mood of a person, with effects that may range from an extreme of excitement to a diamterically opposed extreme of relaxation. The same applies to colours, to the point that a specific colour, or homogeneous range of colours, is usually recommended for each particular living space or room owing to its better suiting to the function of the same space or room.

However, simply combining or matching fragrances, lights and sounds at pleasure, ie. according to personal tastes, in view of enhancing their beneficial effects on the human body is not to be recommended, since selecting the various components in a manner that is not appropriately coordinated and/or balanced might well lead to a mutual neutralization of the properties or even negative results altogether.

US-A-3,585,991 discloses an apparatus belonging to the mentioned state of the art, which is useful to implement a psychophysiosonic system with multisensory aids. It is provided with a control panel, wherein manual switches are individually operable at will of the user.

It therefore is a main purpose of the present invention to provide an apparatus comprising a bathtub and a shower column as defined in claim 1 for the treatment of the human body which makes use of particular combinations of light beams and music sounds with mixtures of aromatic essences in order to bring about definite sensations of psychophysical well-being in a person.

A further purpose of the present invention is to provide a method to be carried out by means of such an apparatus.

The features and advantages of the invention will be more readily understood from the description that is given below by way of non-limiting example with reference to the accompanying drawing, in which:

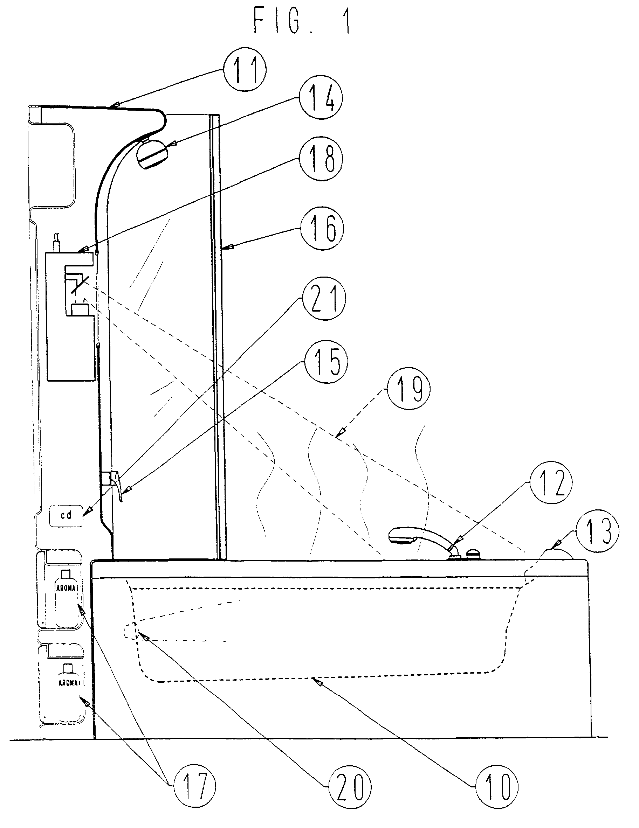

- Figure 1 is a schematical side view of an apparatus for the implementation of the method according to the present invention;

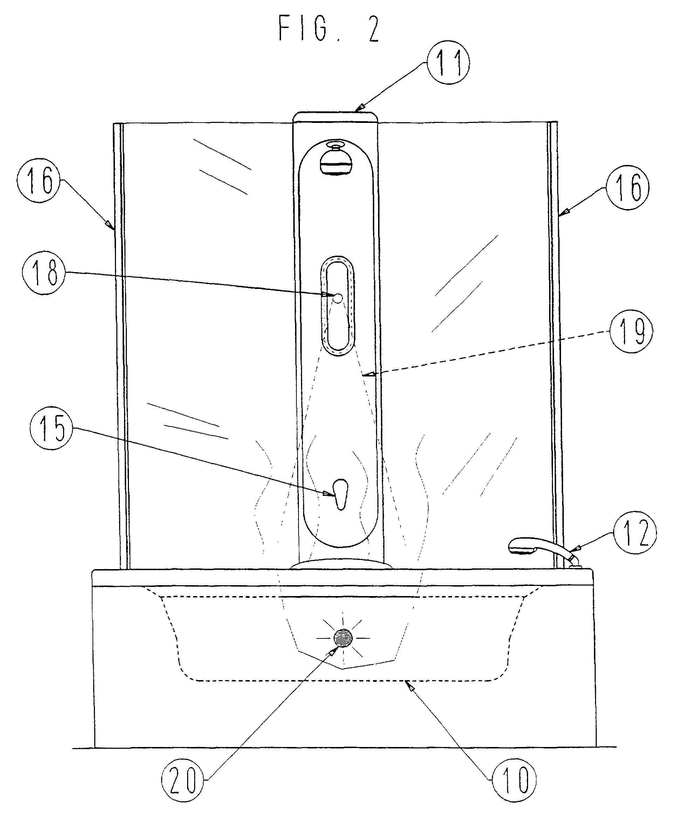

- Figure 2 is a schematical front view of the apparatus of Figure 1; and
- Figure 3 is a schematical top view of the apparatus of Figures 1 and 2.

The apparatus illustrated in the Figures is essentially constituted by a bathtub 10 combined with a column-type shower 11, of a generally known type.

The bathtub 10 is provided with the usual water control means 12, such as knobs, buttons and the like, to control both delivery and draining of hot and cold water. The bathtub 10 is further provided with a head-resting pad 13.The column 11 is provided at its upper portion with a preferably swivelling water delivery head 14 for the delivery of hot and cold water under the control of appropriate control means 15. Furthermore, the column 11 is contained between walls 16 which are preferably of semitransparent toughened glass.

According to the present invention, the apparatus formed by the bathtub 10 and the column-type shower 11 is provided with means for the addition of aromatic substances to the water, as well as means for the generation of light beams of different colours, and means for the diffusion of music sounds, which are all directed towards the body of the person lying in the bathtub 10. As this is better explained further on in this description, the operation of all these means must be appropriately combined and coordinated in order to enable particular beneficial effects to be obtained for the person who is using the apparatus.

The means for the addition of aromatic substances are constituted by reservoirs 17 which contain such substances and are fitted in appropriate seats that are preferably provided in the lower portion of the column 11 and are closed by lids. Said reservoirs 17 are connected in a per sè known manner, via conduits provided with valves and possible metering pumps, to the normal water dispensers associated to the controls 12 and located in the bathtub 10 and the column 11. The aromatic substances used in this connection are in particular essences or essential oils, ie. mixtures of aromatic substances produced by different plants. These essences evaporate in air and are soluble in oils and alcohols, whereas they are insoluble in water, to which they however convey the fragrance and the properties that prove beneficial to the human body. Such essences are very delicate and must be handled correctly in order to prevent them from spoiling and losing their properties. In particular, they must be kept, ie. stored in a way in which they are duly protected from air and light. Such essences have properties that are largely known since many millennia now, as is on the other hand a largely known fact that they are absorbed by the human body through both the respiratory tract (olfactory stimulations pass directly on to the cerebral cortex) and the skin (by passing into the blood and diffusing into the cells of the organism).

The means for the generation of light beams of different colours are constituted by a light projector 18 that is preferably installed in the column 11 of the shower and is provided in a per sè known manner with selectable coloured filters for producing light beams 19 of an appropriate colour to be directed towards the body of the person lying in the bathtub 10. The light beam 19 is controlled, with per sè known means, so as to be caused to move according to alternate rectilinear and rotary patterns, thereby brushing the body of the person according to pre-determined programmes, schemes and paths. Furthermore, an underwater light 20 is provided in a fixed position inside the bathtub 10 to issue a light beam of a rose colour. This light 20 switches on automatically at the beginning of the treatment cycles and stays on throughout the same cycle to floodlight the body of the person longitudinally.

The means provided to diffuse music sounds are preferably constituted by a CD-ROM player 21, which is installed in the column 11 and is connected to submersed loudspeakers 22 mounted on the wall of the bathtub 10. The sound issued by these loudspeakers, further to being audible in the ambient, generates mainly vibrations 23 that pass on to the water contained in the bathtub and act directly on the surface of the body immersed therein.

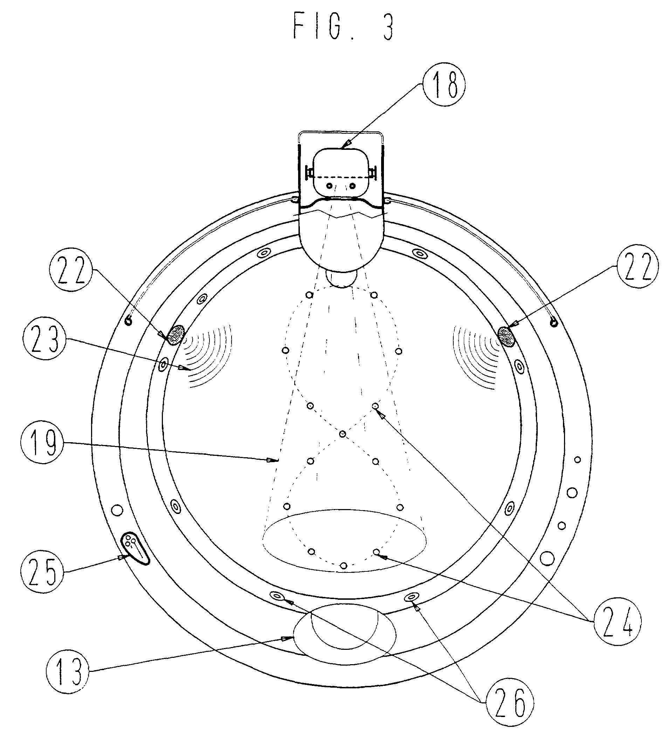

The invention has been described in such a manner as to be capable of being readily implemented by anyone skilled in the art with the use of readily available component parts. It will of course be appreciated that the actual arrangement of the various component parts may be appropriately varied, for instance in accordance with the particular type of bathtub used, ie. with or without shower. Furthermore, the apparatus must be provided with a programme sequence control or timing device 25 (preferably arranged on the rim of the bathtub 10 - see Figure 3) that enables the operation of the various functions provided by the method to be appropriately co-ordinated. Such a sequence control or timing device can be of any suitable type and is anyway easily implementable with readily available electronic techniques.

In particular, the bathtub 10 can be a whirlpool-type tub and, therefore, be provided with spouts or ports 26 for the delivery of water/air mixtures thereinto, so as this is generally known in the art.

The method according to the present invention, which is carried out with the above described apparatus, can be summarized into following phases.

At the beginning, the bathtub 10 is filled with water up to the desired level and at the preferred temperature. The desired programme is then selected by actuating the corresponding push-button or knob included in the control panel or unit 25. The programme is defined, for instance, on the basis of the colour of the light beam 19 that illuminates immediately. Each programme, as defined by a colour, corresponds to a definite treatment with particular beneficial effects on the human body. For instance, the programme defined by the colour green has a bracing, invigorating effect, whereas the one defined by the colour blue has a relaxing effect.

At the same time as the coloured light beam 19 goes on, also the underwater floodlight 20 illuminates and the music sound, which is associated in a programmed manner to the selected colour, starts to be diffused.

It is only after a pre-set time, eg. one minute, that the particular essence co-ordinated with the selected programme starts to be drawn off its resdervoir 17 and mixed with the water in the bathtub 10. Such a delay in the addition of the essence to the water in the bathtub is provided in order to enable the user to possibly change or correct the selection of the programme before different fragrances can mix in the water and, therefore, force the user to first empty and then thoroughly rinse the bathtub.

Each treatment programme has a pre-set duration, eg. twenty minutes, and throughout such a duration of the programme, the coloured light beam 19 is caused to swing along the body of the person in the bathtub with a cyclic and continuous motion. In particular, said light beam is caused to move with an alternate rectilinear and rotary motion. For instance, the beam 19 is caused to oscillate longitudinally for five minutes with a to-and-fro movement from the head to the feet and vice-versa, and then is caused to swing for a further five-minute period with a rotary motion; finally, the alternate longitudinal motion is repeated for ten minutes.

The various programmes are pre-defined by people who have special skills and knowledge in the particular effects generated by the different colours, sounds and fragrances on the human body. The programmes themselves cannot be modified by the user, who is therefore only able to select the programme that offers the most appropriate colour-sound-aroma combination in view of obtaining the desired effect.

The apparatus according to the present invention may be improved so as to produce a further particular beneficial effect. In fact, the bathtub 10 may be provided with a plurality of nozzles 24 for the injection of air. Such nozzles (Figure 3) are arranged on the bottom of the bathtub according to a 8-shaped configuration and may be supplied in a sequence so as to direct their respective jets against the body of the person according to a fixed pattern or course that substantially follows the contour of the body. Such a sequential feeding of the nozzles 24 is co-ordinated with the movement of the light beam 19, so as to namely allowing the air jets to act on the different parts of the body at the same time as the coloured light beam, as well as the sound and the aroma that are co-ordinated therewith. It has been found experimentally that such a particular combined action enhances the desired effect.

As far as the particular combinations of colours, sounds and aromas are on the contrary concerned, these must be studied and defined by the respective specialists and may be continuously modified and improved in accordance with the outcome of the experimental work being done. To merely orientative purposes, with reference to the afore described example of a treatment cycle, it can be stated that, to obtain a bracing or invigorating effect, a programme requires the use of a green-coloured light beam combined with a music of the ''ocean-surf" type (ie. conveying the sensation of the sound of the ocean waves) and an aroma constituted by the essences of lemon, mint and geranium.

## Claims

1. Apparatus for bringing about beneficial effects on the human body comprising a bathtub (10), said apparatus being provided with means (17) for the addition of aromatic substances to the water and with programmable control means (25) to automatically co-ordinate the operation of said means (17) with the operation of means (18) generating light beams and comprising a first floodlight which is adapted to illuminate the human body, and the operation of means (22) for the diffusion of music sounds the apparatus being **characterised in that** it comprises a shower column (11) and **in that** said first floodlight (18) is movable, is installed in said shower column (11) and is operated by actuating means so as to cause the light beam (19) to swing with a cyclic and continuous movement along the human body.

2. Apparatus according to claim 1, **characterised in that** said means (18) generating light beams and said means (22) diffusing music sounds are activated simultaneously in advance of said means (17) for the addition of aromatic substances to the water.

3. Apparatus according to claim 1 or 2, **characterised in that** said means (18) generating light beams comprise a second fixed floodlight (20) that is adapted to illuminate the water with a light beam of a rose colour.

4. Apparatus according to any of the preceding claims, **characterised in that** nozzles (24) are provided on the bottom of the bathtub (10) in a 8-shaped arrangement, which are fed sequentially so as to issue air jets in a progressive manner against the various parts of the human body.

5. Method for bringing about beneficial effects on the human body through the use of a combination of external actions so as to produce definite sensations of well-being, said method being carried out by means of the apparatus according any of the preceding claims.

## Patentansprüche

1. Vorrichtung zur Verursachung günstiger Auswirkungen auf den menschlichen Körper, die eine Badewanne (10) umfasst, wobei die Vorrichtung mit Einrichtungen (17) zum Zusetzen aromatischer Substanzen zu dem Wasser sowie mit einer programmierbaren Steuereinrichtung zum automatischen Koordinieren der Funktion der Einrichtung (17) mit der Funktion einer Einrichtung (18), die Lichtstrahlen erzeugt und einen ersten Scheinwerfer umfasst, der so eingerichtet ist, dass er den menschlichen Körper beleuchtet, und mit der Funktion einer Einrichtung (22) zum Ausstrahlen musikalischer Klänge versehen ist, wobei die Vorrichtung **dadurch gekennzeichnet ist, dass** sie eine Duschsäule (11) umfasst, und dadurch, dass der erste Scheinwerfer (18) beweglich ist, in der Duschsäule (11 ) installiert ist und durch eine Betätigungseinrichtung betätigt wird, um zu bewirken, dass der Lichtstrahl (19) mit einer zyklischen und kontinuierlichen Bewegung über den menschlichen Körper geschwenkt wird.

2. Vorrichtung nach Anspruch 1, **dadurch gekennzeichnet, dass** die Einrichtung (18), die Lichtstrahlen erzeugt, und die Einrichtung (22), die musikalische Klänge ausstrahlt, simultan vor der Einrichtung (17) zum Zusetzen aromatischer Substanzen zu dem Wasser aktiviert werden.

3. Vorrichtung nach Anspruch 1 oder 2, **dadurch gekennzeichnet, dass** die Einrichtung (18), die Lichtstrahlen erzeugt, einen zweiten stationären Scheinwerfer (20) umfasst, der so eingerichtet ist, dass er das Wasser mit einem rosenfarbigen Lichtstrahl beleuchtet.

4. Vorrichtung nach einem der vorangehenden Ansprüche, **dadurch gekennzeichnet, dass** Düsen (24) am Boden der Badewanne (10) in einer 8-förmigen Anordnung vorhanden sind, die aufeinanderfolgend gespeist werden, um fortlaufend Luftstrahlen auf die verschiedenen Teile des menschlichen Körpers abzugeben.

5. Verfahren zum Verursachen günstiger Auswirkungen auf den menschlichen Körper durch den Einsatz einer Kombination äußerer Vorgänge, um bestimmte Empfindungen des Wohlbefindens zu erzeugen, wobei das Verfahren mittels der Vorrichtung nach einem der vorangehenden Ansprüche ausgeführt wird.

## Revendications

1. Dispositif pour susciter des effets bienfaisants sur le corps humain, comprenant une baignoire (10) et une colonne de douche (11), ledit dispositif étant pourvu de moyens (17) pour ajouter des essences aromatiques dans l'eau, ainsi que moyens de contrôle programmables (25) pour coordonner automatiquement le fonctionnement desdits moyens (17) avec le fonctionnement de moyens (18) produisant de faisceaux lumineux et comprenant un premier projecteur qui est apte à éclairer le corps humain, et le fonctionnement de moyens (22) pour diffuser de sons musicaux, ledit dispositif étant **caractérisé en ce qu**'il comprend une colonne de douche (11), et en ce que ledit premier projecteur (18) est mobile, est installé dans ladite colonne de douche (11) et est actionné par de moyens de commande de façon telle à faire osciller le faisceau lumineux (19) avec un mouvement cyclique et continu au long du corps humain.

2. Dispositif selon la revendication 1, **caractérisé en ce que** lesdits moyens (18) produisant les faisceaux lumineux et lesdits moyens (22) diffusant les sons musicaux sont activés simultanément avant lesdits moyens (17) pour ajouter les essences aromatiques dans l'eau.

3. Dispositif selon la revendication 1 ou 2, **caractérisé en ce que** lesdits moyens (18) produisant les faisceaux lumineux comprennent un deuxième projecteur fixe (20) qui est apte à éclairer l'eau avec un faisceau de lumière ayant une couleur rose.

4. Dispositif selon l'une quelconque des revendications précédentes, **caractérisé en ce que** des gicleurs (24) sont pourvus dans le fond de la baignoire (10) avec une disposition en forme de 8, lesdits gicleurs étant alimentés en séquence de façon à émettre des jets d'air de façon progressive contre les différentes parties du corps humain.

5. Procédé pour susciter des effets bienfaisants sur le corps humain par l'utilisation d'une combinaison d'actions externes de façon à produire des sensations bien définies de bien-être, ledit procédé étant exécuté au moyen d'un dispositif selon l'une quelconque des revendications précédentes.
